# EUROPÄISCHE PATENTSCHRIFT

(11) **EP 0 995 096 B1**
(45) Veröffentlichungstag und Bekanntmachung des Hinweises auf die Patenterteilung: **30.10.2002**
(21) Anmeldenummer: 98946237.9
(22) Anmeldetag: 15.07.1998
(51) Int. Cl.: G01N 1/00

(54) **MAGNETSTIFT ZUR KONZENTRIERUNG UND SEPARIERUNG VON PARTIKELN**
MAGNETIC PIN FOR CONCENTRATING AND SEPARATING PARTICLES
CRAYON MAGNETIQUE POUR LA CONCENTRATION ET LA SEPARATION DE PARTICULES

(30) Priorität: 16.07.1997 DE 19730497
(43) Veröffentlichungstag der Anmeldung: 26.04.2000
(73) Patentinhaber: Heermann, Klaus-Hinrich, Dr., D-3400 Göttingen (DE)
(72) Erfinder: Heermann, Klaus-Hinrich, Dr., D-3400 Göttingen (DE)
(74) Vertreter: Schüssler, Andrea, Dr.
(86) Internationale Anmeldenummer: DE9802014
(87) Internationale Veröffentlichungsnummer: WO99004239

(56) Entgegenhaltungen:
- WO-A-87/05536
- WO-A-94/18565
- WO-A-96/12959
- US-A- 2 417 762
- PATENT ABSTRACTS OF JAPAN vol. 098, no. 003, 27. Februar 1998 & JP 09 304385 A (SHIONOGI &CO LTD; SODA NIKKA KK), 28. November 1997

## Beschreibung

Die vorliegende Erfindung betrifft einen Magnetstift zur Konzentrierung und Separierung von Partikeln.

Die heutigen Nachweisverfahren für Nukleinsäuren, wie Polymerase-Chain-Reaction (PCR), Ligase-chain-reaction (LCR), Nucleic Acid Sequence Based Amplification (NASBA), Strand Displacement Amplification (SDA) können durch Amplifikation von Nukleinsäuren mit extremer Empfindlichkeit durchgeführt werden. Durch diese Empfindlichkeit sind die Verfahren sehr anfällig gegen Kontaminationen. Die Kontaminationsgefahr wächst dabei mit der wachsenden Anzahl von Schritten und den auszuführenden Manipulationen, wie Zentrifugier- und Pipettierschritten. So sollte den Experimentatoren daran gelegen sein, die Anzahl der Schritte und Manipulationen möglichst gering zu halten. Dies ist jedoch oft problematisch, da für unterschiedliche Schritte unterschiedliche Probenvolumina eingehalten werden müssen und zwischendurch eine Konzentrierung des Probenvolumens notwendig ist. So besteht der Bedarf nach einer Möglichkeit bei den oben genannten Verfahren Manipulationsschritte, insbesondere Zentrifugationsschritte, einzusparen, indem die zu bestimmende Probe aus den verschiedenen Gefäßen, wie Cups oder Mikrotiterplatten, direkt herausgeholt werden kann und überführt werden kann. Dieser Bedarf Proben auf einfache Weise von einem großen Volumen in ein kleineres Volumen zu überführen bzw. auf einfache Weise die Probengefäße wechseln zu können, beschränkt sich natürlich nicht nur auf das Gebiet der Nukleinsäuretechnik, sondern besteht in der gesamten (Bio)chemie, wo die Aufkonzentrierung von Proben notwendig ist.

Die WO 94/18565 beschreibt einen Magnetstift mit einer Hülle zur Anlagerung von Partikeln. Der Magnetstift kann in einer Hülle verschoben werden. Die Hülle weist eine Haltevorrichtung auf, mit der sie mittels eines Automaten zwischen Probengefäßen versetzt werden kann. Aufgrund der einfachen Verschieblichkeit des Magnetstifts in der Hülle kann die Vorrichtung nicht am Magnetstift versetzt werden, sondern muß immer an der Hülle gegriffen werden.

Die Aufgabe der vorliegenden Erfindung besteht deshalb in der Bereitstellung einer Vorrichtung mit der Zielmoleküle schnell und auf einfache Weise aus Gefäßen herausgeholt und in andere Gefäße überführt werden können, wodurch Manipulationsschritte, wie Zentrifugationsschritte, eingespart und in kleinen Volumina gearbeitet werden kann.

Gelöst wird diese Aufgabe durch einen Magnetstift gemäß Patentanspruch 1. Vorteilhafte Ausgestaltungen ergeben sich aus den Unteransprüchen. Ansprüche 10, 11 und 14 beziehen sich auf eine Verwendung des Magnetstiftes.

Um mit dem erfindungsgemäßen Magnetstift einen Vorteil bei den oben genannten Verfahren zu erreichen, muß natürlich eine magnetische Markierung der Zielmoleküle vorliegen. Wie dies erreicht werden kann, ist dem Fachmann bekannt. Dies wird beispielsweise dadurch erreicht, daß die Zielmoleküle selbst magnetische Eigenschaften haben oder an "magnetic beads" gebunden sind. Eine andere Möglichkeit, falls es sich um ein Nachweisverfahren handelt, ist es, Streptavidin an Eisen zu koppeln und damit die Zielmoleküle zu binden, die ihrerseits mit Biotin markiert sind.

In einem beispielhaften Verfahrens, bei dem der erfindungsgemäße Magnetstift zum Einsatz kommt, wird das Probenmaterial, z.B. Serum, Plasma, Vollblut, Zellen oder Gewebe, einem chemischen oder chemisch-enzymatischen Aufschluß bzw. einer Denaturierung unterworfen. Diese Lösung enthält gleichzeitig ein erstes Nukleinsäure-Oligonukleotid (Primer), das als Sonde an die Zielmoleküle hybridisiert. Dieses Oligonukleotid ist kovalent mit einem Biotinmolekül verbunden, das mit hoher Affinität von Streptavidin gebunden werden kann. Dieses Streptavidin ist seinerseits wiederum an Partikel gebunden, die Eisen beinhalten. Weil die Streptavidin-Partikel nicht in ein denaturiertes Probengemisch gegeben werden können, da sie ansonsten gleichfalls denaturieren würden und ihre Bindungseigenschaften an Biotin verlieren würden, muß das Probengemisch verdünnt werden, was zu einer Zunahme des Probenvolumens führt. Dieses große Probenvolumen wirkt sich allerdings negativ auf sich anschließende Schritte, wie z.B. Amplifikationen, aus. So werden die entstandenen Konjugate (Probe-Oligonukleotid-Biotin-Streptavidin-Eisen) mittels des erfindungsgemäßen Magnetstifts in ein anderes Gefäß überführt, wo dann in einem sehr viel kleineren Volumen die weiteren Schritte des Verfahrens stattfinden können.

Die Erfindung wird weiter anhand der Figuren beschrieben, die zeigen:
- Fig. 1a:: Einzelkomponenten eines erfindungsgemäßen Magnetstifts 8
- Fig. 1b:: Zusammengebauter erfindungsgemäßer Magnetstift 8
- Fig. 2:: Überführung von Partikeln aus einem großen Probengefäß in ein kleines Probengefäß
- Fig. 3a:: Einzelkomponenten eines weiteren erfindungsgemäßen Magnetstifts 22
- Fig. 3b:: Zusammengebauter erfindungsgemäßer Magnetstift 22

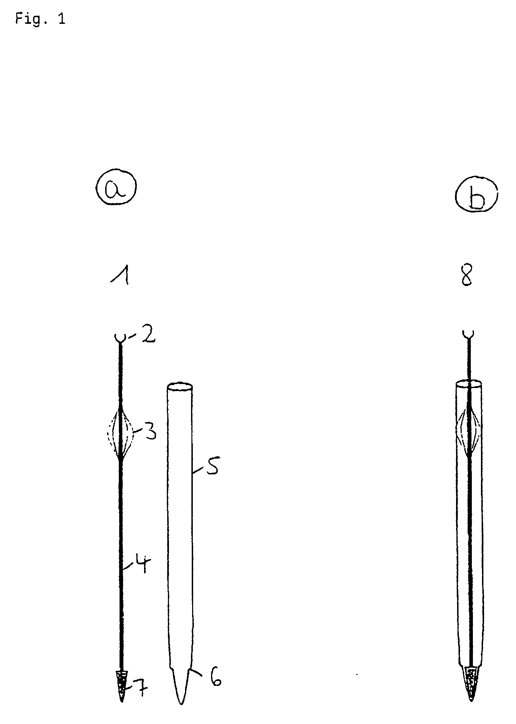

Die Einzelkomponenten einer Ausführungsform eines erfindungsgemäßen Magnetstifts sind in Fig. 1a gezeigt. Der Magnetstift 1 weist an seinem hinteren Ende einen Greifadapter 2 auf. Dieser ist so ausgestaltet, daß er von einem kommerziellen Pipettierautomaten gegriffen und bedient werden kann. Der Greifadapter 2 ist dabei bevorzugt aus einem verformbaren Material, wie z.B. Gummi. Weiter weist der Magnetstift 1 einen Verbindungsstift 4 mit einer Ausbuchtung 3 auf, um für den Pipettierautomaten einen Angriff zu bilden. Weiter dient die Ausbuchtung dazu, um eine Hülle 5 festzuhalten. Diese Hülle 5 ist dünnwandig (Wanddicke ca. 0,2 bis 0,7 mm, bevorzugt 0,3 mm) und besteht vorzugsweise aus polymeren Kunststoffen (z.B. Polypropylen) bzw. einem nicht-magnetisierbaren, jedoch leitfähigen, Material, z.B. Kunststoffen mit Graphitanteil. Die Hülle 5 ist am unteren Ende vorzugsweise konisch geformt und weist eine Verengung 6 auf, die vorzugsweise so an Probengefäße angepaßt ist, daß sie bei geringgradigem Flüssigkeitsvolumen (z.B. 25 µl in einem PCR-Cup) gerade noch in einem gefüllten Probengefäß benetzt wird. Der Verbindungsstift 4 sollte aus einem nicht-magnetischen Material, wie z.B. Aluminium sein. Am vorderen Ende weist der Magnetstift einen vorzugsweise konisch geformten Magnetkopf 7 auf. Dafür kommt jeder übliche Permanentmagnet in Frage.

In Fig. 1b ist ein mit der Hülle 5 versehener Magnetstift 1 als erfindungsgemäßer Magnetstift 8 gezeigt.

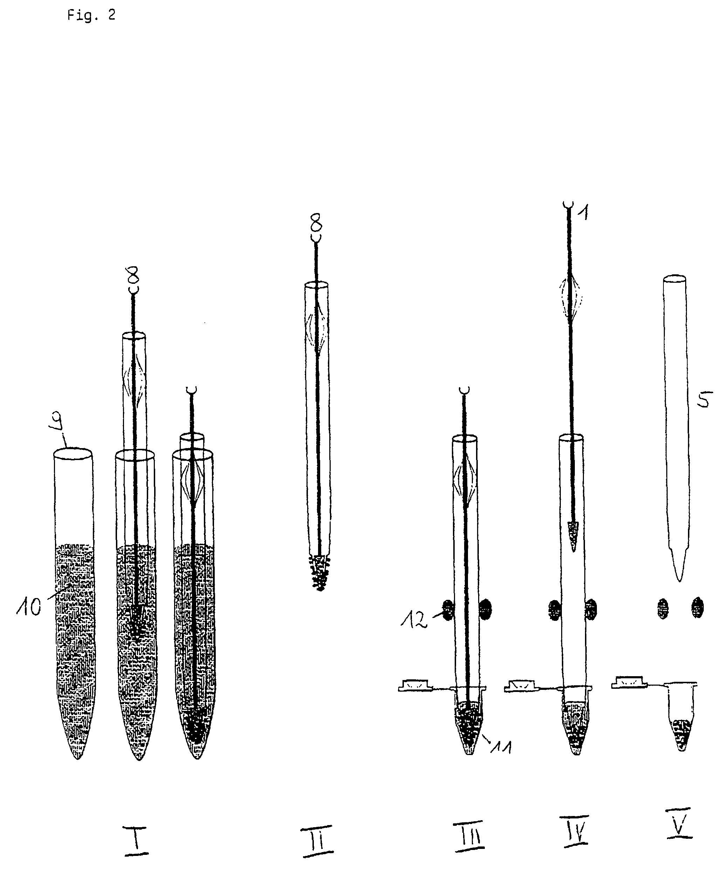

Die Konzentrierung des in den Probengefäßen vorhandenen magnetisch-markierten Probenmaterials wird vorzugsweise mit dem in der Hülle befindlichen Magnetstift 8 ausgeführt und verläuft vorzugsweise wie in Fig. 2 gezeigt.
I) In ein großes Probengefäß 9 mit sich in einer Flüssigkeit befindlichen magnetisch markierten Partikeln 10 wird der Magnetstift mit Hülle 8 eingetaucht. Stift und Hülle werden langsam nach unten bewegt. Die Partikel werden vom Magnetkopf des Magnetstiftes angezogen.
II) Der Magnetstift mit Hülle 8 wird langsam wieder aus dem Probengefäß gezogen, sodaß nur geringe Flüssigkeitsrückstände an der Hülle haften bleiben. Gegebenenfalls können die anhaftenden Partikel in einem Waschgefäß weiteren Behandlungen unterzogen werden, ohne daß es zu einem Verlust der anhaftenden Partikel kommt.
III) Die Spitze des Magnetstiftes einschließlich der sie umgebenden Hülle wird in ein kleineres Probengefäß 11 getaucht. Durch die Anpassung des Magnetkopfes 7 einschließlich der umgebenden Hülle an die Form des Probengefäßes ist nur ein geringes Flüssigkeitsvolumen notwendig, um die Hülle der gesamten Spitze zu benetzen. Die Hülle wird in einem Ständer 12 fixiert.
IV) Der Magnetstift 1 wird durch eine schnelle Bewegung aus der Hülle herausgezogen. Die Partikel lösen sich idealerweise durch die wegfallende Magnetkraft von der Hülle und verbleiben im Probengefäß 11. Sollte dies nicht genügen, werden sie von einem weiteren außen unter dem Probengefäß befindlichen Magneten nach unten gezogen.
V) Die Hülle 5 wird vorsichtig aus dem Probengefäß 11 entfernt.

Ergebnis: Die Partikel befinden sich nun in einem kleineren Volumen in einem anderen Probengefäß.

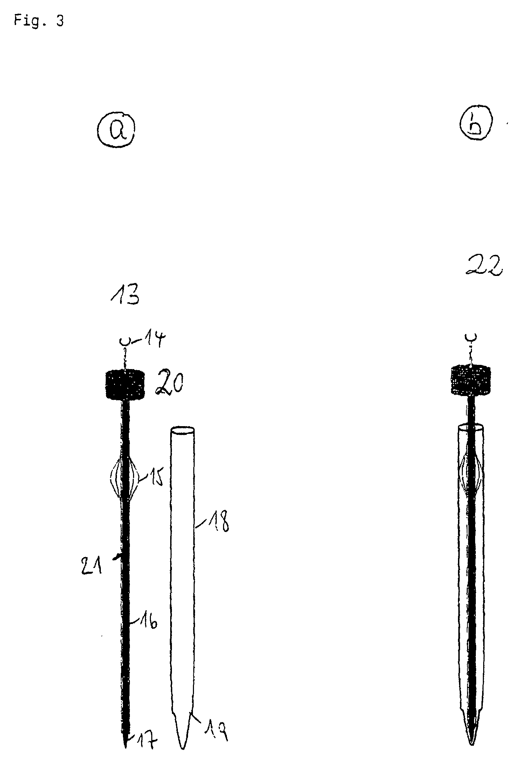

Eine andere bevorzugte Ausführungsform eines erfindungsgemäßen Magnetstifts ist in Fig. 3 gezeigt. Die Einzelkomponenten eines solchen Magnetstifts sind in Fig. 3a gezeigt. Ebenso wie in Fig. 1 weist der Magnetstift einen Greifadapter 14, eine Ausbuchtung 15, einen Verbindungsstift 16, eine Hülle 18 und eine Verengung 19 auf. Die oben erwähnten Produkt- und Größenparameter für diese Bestandteile gelten auch für die Ausführungsform gemäß Fig. 3. Bei der in Fig. 3 gezeigten Ausführungsform gehen die magnetischen Kräfte, die an der Spitze 17 gebündelt austreten sollen, von einem weiter oben angebrachten größeren Permanentmagneten 20 aus und werden durch z.B. einen Eisenkern, in die Spitze 17 geleitet. In diesem Fall kann das direkt aus dem Magneten austretende Magnetfeld durch eine metallische Hülle 21, wie eine Eisenhülle, abgeschirmt sein. Der Magnet 20 kann sich auf dem Verbindungsstift 16 auf jeder beliebigen Höhe befinden. Bevorzugt befindet er sich jedoch nahe dem Greifadapter, da er weiter unten beim Eintauchen des Magnetstifts in ein Probegefäß störend sein kann. Die Spitze 17 besteht aus einem magnetisierbaren Material bzw. kann selbst magnetische Eigenschaften haben, d.h. die Fortführung des Eisenkerns sein.

In Fig. 3b ist ein mit der Hülle 18 versehener Magnetstift 13 als erfindungsgemäßer Magnetstift 22 gezeigt.

Erfindungsgemäß soll die magnetische Kraft durch die Vermittlung anderer geeigneter Substanzen die Separation von Zielsubstanzen bewirken. Deshalb ist nicht der Magnet an sich entscheidend, sondern seine Form und seine Einrichtung. Die Form des Magneten beeinflußt einerseits ganz entscheidend den Verlauf des Magnetfeldes und damit seine Kraft, andererseits muß die Formgebung in diesem Fall sehr genau mit der Zielanwendung in Einklang stehen. In einer bevorzugten Ausführungsform erfolgt das Einsammeln von magnetischen Partikeln aus Lösungen (wie Serum) auf einem möglichst kleinen Areal (Spitze des Magnetstiftes). Dabei soll die magnetische Kraft möglichst hoch sein, damit das Ernten der Magnetpartikel über einen geeigneten Gefäßquerschnitt erfolgen kann. Durch ein langsames Absenken des Magnetstiftes innerhalb des Gefäßes (z.B. Serum-Röhrchens) wird zusätzlich ein Ernten der Magnetpartikel aus einem wesentlich größeren Ausgangsvolumen möglich. Darüberhinaus muß die Formgebung der Magnetspitze darauf angepaßt sein, daß beim Herausziehen des Magnetstiftes möglichst wenig Flüssigkeit an ihm haften bleibt. Gleichzeitig ist bei der Formgebung der Spitze zu berücksichtigen, daß es beim Eintauchen des Stiftes in Lösungen nicht zum Übertritt von kleinsten Anteilen dieser Lösung (Tröpfchen, Aerosole) in die Luft kommen darf. Letztendlich muß die Formgebung des Stiftes auch auf das Zielgefäß adaptiert sein, so daß dort die Vorlage eines möglichst kleinen Flüssigkeitsvolumens ausreicht, um die Stelle der anhaftenden Magnetpartikel vollständig zu benetzen. Die Plastikhülle des Magnetstiftes hat zu diesem Zeitpunkt die Aufgabe, das Abstellen der magnetischen Kraft zu ermöglichen. Um dieses Ziel zu erreichen, wird der Magnetstift zügig aus der Hülle gezogen, worauf die Magnetpartikel von der Außenseite der Hülle abfallen.

So bietet die erfindungsgemäße Ausführungsform mit spitzer Form des Magneten besondere Vorteile beim Herausziehen aus einer Lösung. Über eine Messung der Induktion beim Einsatz leitfähiger Hüllen kann dieser Vorgang zeitlich auch automatisch so gesteuert werden, daß an der aus der Flüssigkeitsoberfläche entfernten Spitze kein Tropfen mehr verbleibt; die Ausgangslösung ist maximal reduziert (Einschritt-Konzentrierung). Dies ist ein wichtiges Detail nicht nur zur Verhinderung von Kontaminationsgefahren beim Bewegen über anderen Proben, sondern auch für die Abtrennung und Konzentrierung von Magnetpartikeln, die beim Einsatz einer flachen Spitze so nicht möglich ist. Außerdem ist bei einer spitzen Form des Magneten das Magnetfeld quer ausgerichtet, wodurch eine verbesserte Sammelwirkung eintritt.

Somit können durch eine spitze Ausformung, die an die verwendeten Gefäße anzupassen ist, die Magnetpartikel in äußerst kleinem Flüssigkeitsvolumen abgegeben werden. Verwendet man ein entsprechendes Gefäß auf der Aufnahmeseite, kann eine Magnetspitze auch zum Übertragen von Substanzen und Entfernen von Verunreinigung durch Waschen aus einem sehr kleinen Ausgangsvolumen benutzt werden.

Der erfindungsgemäße Magnetstift hat weiter den Vorteil, daß die Zielmoleküle der Probe auf einfache Weise ankonzentriert und separiert werden. Weiter ist dies kostengünstig, da die Hülle aus billigem Material ist und verworfen werden kann bzw. gespült werden kann, während der eigentliche Magnetstift eine lange Lebensdauer hat und ohne weitere Reinigung erneut eingesetzt werden kann, da er ideaterweise keinen Kontakt mit der Probe hat.

## Patentansprüche

1. Magnetstift (8, 22) zur Konzentrierung von Partikeln, aufweisend
einen Verbindungsstift (4, 16) mit einem hinteren Ende und einem vorderen Ende (7, 17);
zumindest einen Magneten (7, 20), der so am Verbindungsstift (4, 16) angeordnet ist, daß am vorderen Ende (7, 17) ein Magnetfeld konzentriert austritt, und
eine Hülle (5, 18) , in die der Verbindungsstift (4, 16) einführbar ist;
**dadurch gekennzeichnet, daß**
ein Greifadapter (2, 14) zur Bedienung des Magnetstifts (8, 22) durch einen Automaten am hinteren Ende angeordnet ist;
der Verbindungsstift (4, 16) eine Ausbuchtung (3, 15) zum Festhalten der Hülle (5, 18) aufweist; und
die Hülle (5, 18) assoziiert mit dem oder getrennt vom Verbindungsstift beweglich ist.

2. Magnetstift nach Anspruch 1, wobei der Greifadapter (2, 14) aus einem verformbaren Material ist.

3. Magnetstift nach Anspruch 1 oder 2, **dadurch gekennzeichnet, daß** der Greifadapter so ausgestaltet ist, daß er von einem kommerziellen Pipettierautomaten gegriffen und bedient werden kann.

4. Magnetstift nach einem der Ansprüche 1 bis 3, wobei der Verbindungsstift (4, 16) aus einem nicht magnetischen Material ist.

5. Magnetstift nach einem der Ansprüche 1 bis 4, wobei der Magnet (7, 20) aus einem Permanentmagneten ist.

6. Magnetstift nach einem der Ansprüche 1 bis 5, wobei die Hülle (5, 18) aus einem nicht-magnetisierbaren Material ist.

7. Magnetstift nach einem der Ansprüche 1 bis 6, wobei die Hülle (5, 18) aus Polypropylen ist.

8. Magnetstift nach einem der Ansprüche 1 bis 7, wobei die Hülle (5, 18) eine Wanddicke von 0,2 bis 0,7 mm hat.

9. Magnetstift nach einem der Ansprüche 1 bis 8, wobei die Ausbuchtung (3, 15) eine zur Befestigung der Hülle (5, 18) angepaßte Form hat.

10. Verwendung des Magnetstiftes nach einem der Ansprüche 1 bis 9 zum Waschen und zur Konzentrierung und Separierung von Partikeln.

11. Verwendung nach Anspruch 10, wobei die Konzentrierung von Partikeln in einem Amplifikationverfahren für Nukleinsäuren stattfindet.

12. Magnetstift nach Anspruch 1, **dadurch gekennzeichnet, daß** der Verbindungsstift am vorderen Ende den zumindest einen Magneten als Magnetkopf aufweist.

13. Magnetstift nach Anspruch 1, dadurch gekennzeichnst, daß das vordere Ende eine Spitze ist und der Verbindungsstift einen Magneten aufweist, dessen Magnetfeld an der Spitze konzentriert austritt.

14. Verfahren zum Waschen, zur Separierung und Konzentrierung von Biomolekülen unter Verwendung des Magnetstiftes gemäß einem der Ansprüche 1-9, 12 und 13, wobei
- der Magnetstift (8,22) mit Hülle (5,18) in ein Probengefäß eingetaucht wird, in dem sich Biomoleküle befinden,
- der Magnetstift (8,22) mit Hülle (5,18) zusammen mit den an der Hülle (5,18) haftenden Biomolekülen aus dem Probengefäß (9) gezogen und in mindestens ein weiteres Probengefäß (11) eingetaucht wird,
- der Magnetstift (8,22) aus der Hülle (5,18) herausgezogen und anschließend die Hülle aus dem weiteren Probengefäß (11) entfernt wird, wonach sich die Biomoleküle in dem weiteren Probengefäß befinden.

## Claims

1. A magnetic pin (8, 22) for concentrating particles, comprising
a connecting pin (4, 16) having a rear end and a front end (7, 17);
at least one magnet (7, 20) arranged at the connecting pin (4, 16) such that a magnetic field leaves its front end (7, 17) in concentrated fashion,
a case (5, 18) into which the connecting pin (4, 16) can be introduced;
**characterized in that**
a gripping adapter (2, 14) for operating the magnetic pin (8, 22) is arranged at the rear end by means of an automatic;
the connecting pin (4, 16) has a bulge (3, 15) for holding the case (5, 18); and
the case (5, 18) associated with, or separated from, the connecting pin is movable.

2. The magnetic pin according to claim 1, wherein the gripping adapter (2, 14) is of a deformable material.

3. The magnetic pin according to claim 1 or 2, **characterized in that** the gripping adapter is designed such that it can be gripped and operated by a commercial automatic pipetting apparatus.

4. The magnetic pin according to any of claims 1 to 3, wherein the pipetting pin (4, 16) is of a non-magnetic material.

5. The magnetic pin according to any of claims 1 to 4, wherein the magnet (7, 20) is of a permanent magnet.

6. The magnetic pin according to any of claims 1 to 5, wherein the case (5, 18) is of a non-magnetizable material.

7. The magnetic pin according to any of claims 1 to 6, wherein the case (5, 18) is of polypropylene.

8. The magnetic pin according to any of claims 1 to 7, wherein the case (5, 18) has a wall thickness of 0.2 to 0.7 mm.

9. The magnetic pin according to any of claims 1 to 8, wherein the bulge (3, 16) has a shape adapted for fixing the case (5, 18).

10. Use of the magnetic pin according to any of claims 1 to 9 for washing and concentrating and separating particles.

11. Use according to claim 10, wherein particles are concentrated in an amplification method for nucleic acids.

12. The magnetic pin according to claim 1, **characterized in that** the connecting pin has the at least one magnet as a magnetic head at its front end.

13. The magnetic pin according to claim 1, **characterized in that** the front end is a tip and the connecting pin has a magnet whose magnetic field leaves the tip in concentrated fashion.

14. A method of washing, separating and concentrating biomolecules using the magnetic pin according to any of claims 1-9, 12 and 13, wherein
- the magnetic pin (8, 22) with case (5, 18) is immersed in a sample container containing biomolecules,
- the magnetic pin (8, 22) with case (5, 18) and the biomolecules adhering to the case (5, 18) is pulled out of the sample container (9) and immersed in at least one further sample container (11),
- the magnetic pin (8, 22) is pulled out of the case (5, 18) and then the case is removed from the other sample container (11), whereupon the biomolecules are located in the other sample container.

## Revendications

1. Crayon magnétique (8, 22) pour la concentration de particules, qui présente :
un crayon de jonction (4, 16) avec une extrémité arrière et une extrémité avant (7, 17) ;
au moins un aimant (7, 20), qui est disposé sur le crayon de jonction (4, 16) de telle sorte qu'à l'extrémité avant (7, 17), un champ magnétique sort de façon concentrée ; et
une gaine (5, 18), dans laquelle le crayon de jonction (4, 16) peut être introduit ;
**caractérisé**
**en ce qu'**un adaptateur de prise (2, 14) est disposé à l'extrémité arrière pour la manipulation du crayon magnétique (8, 22) par un automate ;
**en ce que** le crayon de jonction (4, 16) présente un évidement (3, 15) pour le maintien de la gaine (5, 18) ; et
**en ce que** la gaine (5, 18) associée au crayon de jonction ou séparée de ce dernier est mobile.

2. Crayon magnétique selon la revendication 1, pour lequel l'adaptateur de prise (2, 14) est en un matériau déformable.

3. Crayon magnétique selon une des revendications 1 ou 2, **caractérisé en ce que** l'adaptateur de prise est conçu de telle sorte qu'il peut être saisi et manipulé par un automate de manipulation de pipette du commerce.

4. Crayon magnétique selon une des revendications 1 à 3, pour lequel le crayon de jonction (4, 16) est en un matériau non magnétique.

5. Crayon magnétique selon une des revendications 1 à 4, pour lequel l'aimant (7, 20) est en un aimant permanent.

6. Crayon magnétique selon une des revendications 1 à 5, pour lequel la gaine (5, 18) est en un matériau non magnétisable.

7. Crayon magnétique selon une des revendications 1 à 6, pour lequel la gaine (5, 18) est en polypropylène.

8. Crayon magnétique selon une des revendications 1 à 7, pour lequel la gaine (5, 18) a une épaisseur de paroi de 0,2 à 0,7 mm.

9. Crayon magnétique selon une des revendications 1 à 8, pour lequel l'évidement (3, 15) a une forme adaptée à la fixation de la gaine (5, 18).

10. Utilisation du crayon magnétique selon une des revendications 1 à 9 pour le lavage et pour la concentration et la séparation de particules.

11. Utilisation selon la revendication 10, pour laquelle la concentration des particules a lieu dans un procédé d'amplification pour des acides nucléiques.

12. Crayon magnétique selon la revendication 1, **caractérisé en ce que** le crayon de jonction présente à l'extrémité avant le au moins un aimant en tant que tête magnétique.

13. Crayon magnétique selon la revendication 1, **caractérisé en ce que** l'extrémité avant est une pointe et que le crayon de jonction présente un aimant dont le champ magnétique sort concentré à la pointe.

14. Procédé pour le lavage, pour la séparation et pour la concentration de molécules biologiques moyennant l'utilisation du crayon magnétique selon une des revendications 1 à 9, 12 et 13, pour lequel
le crayon magnétique (8, 22) avec la gaine (5, 18) est plongé dans une éprouvette, dans laquelle se trouvent des molécules biologiques,
le crayon magnétique (8, 22) avec la gaine (5, 18) et avec les molécules biologiques qui adhèrent à la gaine (5, 18) est retiré de l'éprouvette (9) et est plongé dans au moins une autre éprouvette (11), et
le crayon magnétique (8, 22) est retiré de la gaine (5, 18) et ensuite la gaine est retirée de l'autre éprouvette (11), après quoi les molécules biologiques se trouvent dans l'autre éprouvette.
